(19) Europäisches Patentamt
European Patent Office
Office européen des brevets

(11) **EP 4 651 144 A1**

(12) **EUROPEAN PATENT APPLICATION**

(43) Date of publication:
**19.11.2025   Bulletin 2025/47**

(51) International Patent Classification (IPC):
***G16C 10/00*** *(2019.01)*

(21) Application number: **25176269.6**

(52) Cooperative Patent Classification (CPC):
**G16C 10/00;** G16C 20/90

(22) Date of filing: **14.05.2025**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **17.05.2024   IN 202421039027**

(71) Applicant: **Tata Consultancy Services Limited Maharashtra (IN)**

(72) Inventors:
• **MUKHERJEE, Anirban**
**400096 Mumbai, Maharashtra (IN)**

• **BHANDARY, Soham**
**400096 Mumbai, Maharashtra (IN)**
• **BANERJEE, Ritam**
**400096 Mumbai, Maharashtra (IN)**
• **GOPAL, Ananthakrishna**
**400096 Mumbai, Maharashtra (IN)**
• **SESHADRI, Janani**
**400096 Mumbai, Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR MULTICONFIGURATIONAL DOWNFOLDING OF MOLECULES**

(57) The present invention generally relates to the field of quantum chemistry, and, more particularly, to a method and system for multi configurational downfolding of molecules. Initially a configuration of a molecule is obtained. Then, one or more electron integrals of the molecule are computed partially on the fly on the GPU and tensor factorization of the partially computed electron integrals is performed to obtain tensor factorized representation of the one or more electron integrals. Further, a density matrix of the molecule is computed based on the tensor factorized representation and multi configurational Hamiltonian downfolding is performed on orbitals of the molecule based on the density matrix.

FIG. 2

EP 4 651 144 A1

## Description

CROSS-REFERENCE TO RELATED APPLICATIONS AND PRIORITY

The present application claims priority to Indian application no. 202421039027, filed on May 17, 2024.

TECHNICAL FIELD

The present invention generally relates to the field of quantum chemistry, and, more particularly, to a method and system for multi configurational downfolding of molecules.

BACKGROUND

In industries such as pharmaceutical and material design domains, when a new material is sought with desired properties, or a property of a material needs to be optimized, currently the industry relies on extensive literature review and experiments to find the relevant force fields or use machine learned force fields. These approaches do not consider quantum many body properties which arise from the many body wavefunctions such as: long range electronic correlations or dispersion effects, multistate representations of the electronic system in the ground state, effect of higher order electron-hole clusters, and excited state of the many-body system. Quantum particles screening of molecules and materials requires computing a variety of descriptors such as single point energy, HOMO-LUMO (Highest Occupied Molecular Orbital- Least Unoccupied Molecular Orbital) gap, dipole-moment, partial charges, band structure, binding free energy, trajectories of atoms and so on. In order to compute these descriptors, it is necessary to determine electronic integrals of the molecules which requires extensive computations and memory. Hence, state of the art methods have attempted to approximate the electronic integrals as a combination of tensor factors to make further processing less complex.

Tensor Factorization is a family of methods to decompose higher rank tensors (3, 4-rank) into a sum of product of lower rank tensors. In traditional quantum chemistry approaches, the initial higher rank tensor comprises the following objects: four-rank electron integral tensor, three rank Cholesky tensor or a 3-center 2-electron integral, and four rank coupled cluster excitation amplitude which is the highest rank tensor comprising the similarity transformation. To carry out tensor factorization of these objects, first, the four rank or three rank objects are stored on a Central Processing Unit (CPU). Next one slice along one dimension of the three or four -rank tensor, i.e., a two-rank or three-rank tensor is transferred to a Graphics Processing Unit (GPU) to compute partial component of the least square cost function and update the tensor factors. CPU to GPU memory transfer of the slices is much slower compared to the processing on the GPU. So even if the GPU processing of expensive compute is controlled, the CPU to GPU transfer rate will slow down the entire process of tensor factorization. One of the recent prior arts implements tensor factorization on GPU by performing compression and reconstruction of the full tensor leading to a memory overhead. It is typically restricted to small tensors or large tensors where high accuracy of tensor factors is not needed.

Once tensor factors are determined, Hamiltonian of the molecule is determined and then downfolded to compute desired properties of the molecule. Downfolding is a family of methods that allow electronic Hamiltonians to be solved in a smaller active space. Traditional downfolding approaches make use of both similarity transformation and unitary transformation. To perform downfolding in a standard manner one must choose between a small primary space and a large complementary space. If downfolding is implemented exactly, then the eigenvalues of the downfolded effective Hamiltonian in the smaller primary space via an appropriate transformation will only contain a subset of the eigenvalues of the full space. In practice, however, downfolding is as costly to implement as solving the many-body Hamiltonian for getting eigenvalues. Therefore, solving the downfolded Hamiltonian must include solving the algebraic equations of the transformation parameters which are equivalent to coupled cluster equations with a certain level of accuracy. Some recent downfolding approaches split the transformation operation and exploit sub-algebra of the operators to simplify expressions. The unitary route to downfolding is inspired by the unitary coupled cluster ansatz which must be truncated up to double-commutator level to obtain Double Unitary Coupled Cluster (DUCC) Hamiltonian. In standard downfolding approaches the similarity or unitary transformation operator has $N^2$ singles and $N^4$ doubles excitation operators. The uncontrolled approximation associated with truncating the similarity transformation in the expression of the Bloch equation leads to loss in accuracy of computing the similarity transformations and thereby the Bloch equation.

SUMMARY

Embodiments of the present disclosure present technological improvements as solutions to one or more of the above-mentioned technical problems recognized by the inventors in conventional systems. For example, in one embodiment, quantum simulation method for multi configurational downfolding of molecules performed by a system comprising one or more Central Processing Units (CPUs) and one or more Graphical Processing Units (GPUs) is provided.

The method includes obtaining, via the one or more CPUs, a configuration of a molecule. Further, the method includes iteratively estimating, via the one or more GPUs, tensor factorized representation of one or more electronic integrals ($A$) of the molecule by partially computing the one or more electronic integrals on the fly based on the configuration of the molecule and performing tensor factorization of each of the partially computed one or more electronic integrals to obtain a tensor factorized representation of each of the one or more electronic integrals. The tensor factorized representation comprises a set of tensor factors. Each tensor factor in the set of tensor factors is a two-rank tensor. The method further includes determining, via the one or more GPUs, a density matrix of the molecule by using a Hartree-Fock calculation based on the tensor factorized representation of the one or more electronic integrals. Furthermore, the method includes performing, via the one or more GPUs, multiconfigurational Hamiltonian downfolding on orbitals of the molecule based on the density matrix.

In another aspect, a system for multiconfigurational downfolding of molecules is provided. The system includes: a memory storing instructions; one or more communication interfaces; and one or more hardware processors coupled to the memory via the one or more communication interfaces, wherein the one or more hardware processors comprise one or more Central Processing Units (CPUs) and one or more Graphical Processing Units (GPUs), and wherein the one or more hardware processors are configured by the instructions to: obtain, via the one or more CPUs, a configuration of a molecule. Further, the one or more hardware processors are configured by the instructions to iteratively estimate, via the one or more GPUs, tensor factorized representation of one or more electronic integrals ($A$) of the molecule by partially computing the one or more electronic integrals on the fly based on the configuration of the molecule and performing tensor factorization of each of the partially computed one or more electronic integrals to obtain a tensor factorized representation of each of the one or more electronic integrals. The tensor factorized representation comprises a set of tensor factors. Each tensor factor in the set of tensor factors is a two-rank tensor. The one or more hardware processors are further configured to determine, via the one or more GPUs, a density matrix of the molecule by using a Hartree-Fock calculation based on the tensor factorized representation of the one or more electronic integrals. Furthermore, the one or more hardware processors are configured to perform, via the one or more GPUs, multi configurational Hamiltonian downfolding on orbitals of the molecule based on the density matrix.

In yet another aspect, there are provided one or more non-transitory machine-readable information storage mediums comprising one or more instructions which when executed by one or more hardware processors cause a method for multi configurational downfolding of molecules. The method includes obtaining, via the one or more CPUs, a configuration of a molecule. Further, the method includes iteratively estimating, via the one or more GPUs, tensor factorized representation of one or more electronic integrals ($A$) of the molecule by partially computing the one or more electronic integrals on the fly based on the configuration of the molecule and performing tensor factorization of each of the partially computed one or more electronic integrals to obtain a tensor factorized representation of each of the one or more electronic integrals. The tensor factorized representation comprises a set of tensor factors. Each tensor factor in the set of tensor factors is a two-rank tensor. The method further includes determining, via the one or more GPUs, a density matrix of the molecule by using a Hartree-Fock calculation based on the tensor factorized representation of the one or more electronic integrals. Furthermore, the method includes performing, via the one or more GPUs, multiconfigurational Hamiltonian downfolding on orbitals of the molecule based on the density matrix.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for multi configurational downfolding of molecules, according to some embodiments of the present disclosure.
FIG. 2 is a flow diagram illustrating a method for multiconfigurational downfolding of molecules, according to some embodiments of the present disclosure.
FIGS. 3 to 6 are graphs illustrating cumulative correlation energy at each downfolding step performed by method of FIG. 2 for a plurality of molecules, according to some embodiments of the present disclosure.
FIG. 7 is a graph illustrating correlation between $IC_{50}$ values of a molecule obtained via method of FIG. 2 (X-axis) and via physical experiments (Y-axis), according to some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Conventional methods of tensor factorization are not scalable since electronic integrals to be factorized are first fully stored on a CPU and then parts of it are transferred to GPU for tensor factorization. As the size of molecules increases, the size of electronic integrals increases due to which memory required to store them also increases. This leads to complexity in storage and computations. Further, conventional downfolding methods are conducted by making a choice of a complete active space and carried out in one shot. This initial choice of complete active space is a trial-and-error approach and is by no means automated. Downfolding is done with respect to a single-reference state thereby restricting molecules that do not have strong electronic correlation.

In order to overcome the above-mentioned drawbacks of conventional techniques, embodiments of present disclosure provide a method and system for multi configurational downfolding of molecules. Initially a configuration of a molecule is obtained. Then, one or more electron integrals of the molecule are computed partially on the fly on the GPU and tensor factorization of the partially computed electron integrals is performed to obtain tensor factorized representation of the one or more electron integrals. Further, a density matrix of the molecule is computed based on the tensor factorized representation and multiconfigurational Hamiltonian downfolding is performed on orbitals of the molecule based on the density matrix. Tensor factorization of higher rank tensor into lower-rank tensor factors is performed by introducing an additional auxiliary direction or ghost direction. Each of the lower rank tensor factors is solved by a least square cost function while keeping the other two tensor factors fixed. This is repeated for the other two tensor factors in the product. Finally, this entire alternating least-squares process is repeated for multiple iterations till convergence.

Thus, the method of present disclosure is scalable to larger molecules since the highest rank of the excitation amplitude in the similarity or unitary transformation is a three-rank tensor rather than a four-rank tensor and the three-rank tensor-slices can be computed on-the-fly on the GPU preventing any CPU to GPU transfer. The three-rank tensor is subsequently factorized into a product of three two rank tensors on the GPU. The four-rank tensor is never constructed. Further, Hamiltonian downfolding is performed iteratively wherein at each iteration the highest energy virtual orbital (that is to be decoupled) is kept fixed and with respect to it all the rest configurations from singles and doubles excitations are considered. Due to the recursive downfolding scheme where one spin-orbital is decoupled at each step, the drawback in traditional approaches regarding choosing complete active spaces is eliminated. The downfolding scheme has an exact unitary matrix representation. Therefore, all the ground and excited states obtained from the initial multireference starting point form an orthogonal complete basis. The unitary transformation can be constructed in closed form offering easy access to the many body wavefunctions. These are not possible in the standard downfolding approaches. Combined with tensor factorization and multi configurational downfolding, embodiments of present disclosure solve the Bloch equations with $O(N^3 - N^4)$ complexity compared to $N_c \times O(N^7)$ complexity in conventional approaches. By performing multi configurational downfolding as in present disclosure, one can capture strong electronic correlations that appears in free radicals, oxidation reactions, transition metal complexes, drug metabolism, transition states where relative energies <0.01 Hartree are involved.

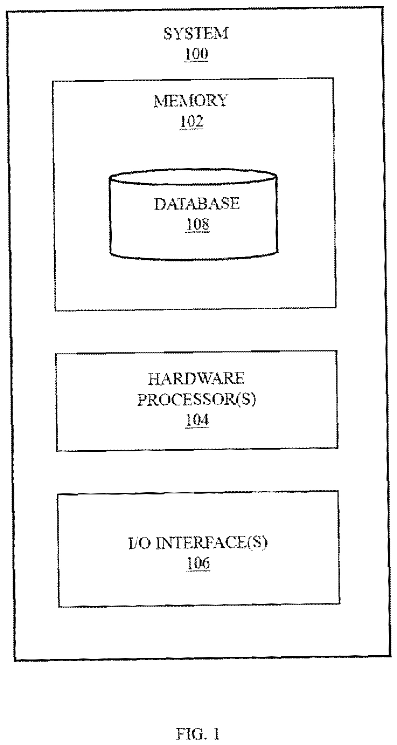

Referring now to the drawings, and more particularly to FIGS. 1 to 7, where similar reference characters denote corresponding features consistently throughout the figures, there are shown preferred embodiments, and these embodiments are described in the context of the following exemplary system and/or method.

FIG. 1 illustrates an exemplary block diagram of a system for multi configurational downfolding of molecules, according to some embodiments of the present disclosure. In an embodiment, the system 100 includes one or more processors 104, communication interface device(s) 106 or Input/Output (I/O) interface(s) 106 or user interface 106, and one or more data storage devices or memory 102 operatively coupled to the one or more processors 104. The one or more processors 104 that are hardware processors can be implemented as one or more microprocessors, microcomputers, microcontrollers, digital signal processors, state machines, logic circuitries, and/or any devices that manipulate signals based on operational instructions. The one or more hardware processors 104 comprise one or more Central Processing Units (CPUs) 104A and one or more Graphical Processing Units (GPUs) 104B (not shown in FIG. 1). Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud, and the like.

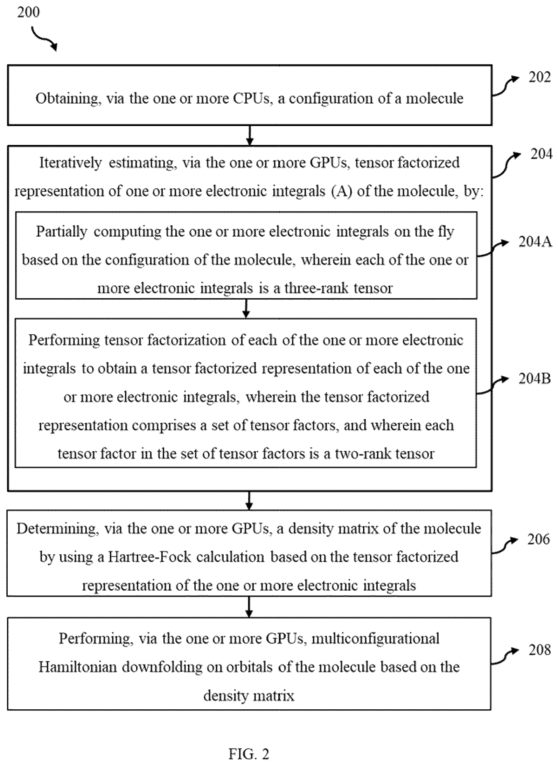

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. The memory 102 may include any computer-readable medium known in the art including, for example, volatile memory, such as Static Random-Access Memory (SRAM) and Dynamic Random-Access Memory (DRAM), and/or non-volatile memory, such as Read Only Memory (ROM), erasable programmable ROM, flash memories, hard disks, optical disks, and magnetic tapes. The database 108 stores information pertaining to inputs fed to the system 100 and/or outputs generated by the system (e.g., at each stage), specific to the methodology described herein. Functions of the components of system 100 are explained in conjunction with the flow diagram depicted in FIG. 2 for multi configurational downfolding of molecules.

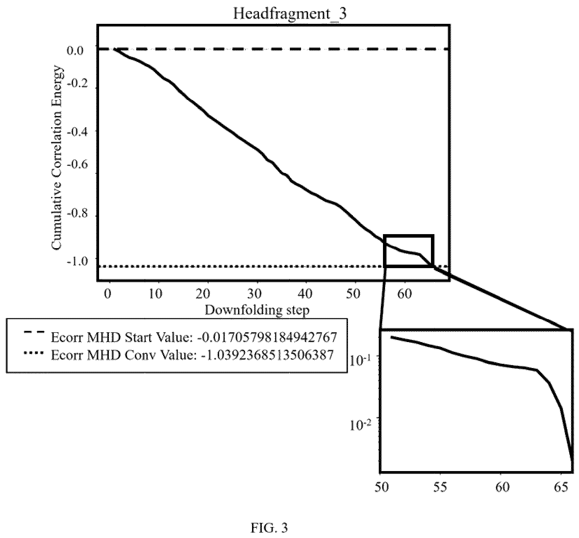

In an embodiment, the system 100 comprises one or more data storage devices or the memory 102 operatively coupled to the processor(s) 104 and is configured to store instructions for execution of steps of the method 200 depicted in FIG. 2 by the processor(s) or one or more hardware processors 104. The steps of the method of the present disclosure will now be explained with reference to the components or blocks of system 100 as depicted in FIG. 1, the steps of flow diagrams as depicted in FIG. 2 and experimental results illustrated in FIGS. 3 to 7. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

FIG. 2 is a flow diagram illustrating a method 200 for multi configurational downfolding of molecules, according to some embodiments of the present disclosure. At step 202, the one or more CPUs 104A are configured to obtain a configuration of a molecule. The configuration of the molecule comprises one of: atomic coordinates, lattice vectors, and basis vectors. For example, configuration of a chemical complex such as amorphous, dust or crystalline material is defined by $x$, $y$, $z$ coordinates of each of its constituent atoms and configuration of a periodic systems is defined by reciprocal lattice vectors. As understood by a person skilled in the art, the periodic systems have repetitive units of molecules, for example, copper oxide layers, drug crystals.

Further, at step 204 of the method 200, the one or more GPUs 104B are configured to iteratively estimate tensor factorized representation of one or more electronic integrals (A) of the molecule. At each iteration, initially the one or more electronic integrals are partially computed on the fly based on the configuration of the molecule at step 204A. In an embodiment, each of the one or more electronic integrals is a three-rank tensor. In an embodiment, the one or more electronic integrals include three-center two-electron integrals and slices of four-center two-electron integrals which are computed using Head-Gordon-Pople recurrence relations using an open-source library of electronic integrals. The shape of three-center two-electron integrals is $(N_{ao}, N_{ao}, N_{aux})$ where $N_{ao}$ is the number of atomic orbital basis function and $N_{aux}$ is the number of auxiliary basis functions. The atomic orbital and auxiliary basis functions are determined based on the chosen library of electronic integrals. The slices of four-center two-electron integrals have a shape of $(N_{ao}, N_{ao}, N_{ao})$. Once the one or more electronic integrals are partially computed, tensor factorization of each of the one or more partially computed electronic integrals is performed to obtain a tensor factorized representation of each of the one or more electronic integrals at step 204B. The tensor factorized representation of A comprises a set of tensor factors (a first tensor factor $X$, a second tensor factor $Y$, and a third tensor factor $Z$) as represented by equation 1. Each tensor factor in the set of tensor factors is a two-rank tensor.

$$A_{ijk} = \sum_a X_{ia} Y_{ja} Z_{ka} \ ..... \ (1)$$

In order to perform tensor factorization, the first tensor factor $X$, the second tensor factor $Y$, and the third tensor factor $Z$ are initialized. In an embodiment, initialization is done by choosing random floating-point numbers that comprise the tensor factors. After initialization, the third tensor factor is updated by: multiplying transpose of the first tensor factor $X$ along a first direction of $A$ to obtain a matrix $B$ (as shown in equation 2); multiplying the matrix $B$ with transpose of the second tensor factor $Y$ along a second direction to obtain a matrix $C$ (as shown in equation 3); determining product of the first tensor factor $X$ with transpose of $X$ as matrix $D$ (as shown in equation 4) and product of the second tensor factor $Y$ with transpose of $Y$ as matrix $E$ (as shown in equation 5); performing Hadamard product of the matrix $D$ and the matrix $E$ to obtain a matrix $F$ (as shown in equation 6); and multiplying inverse of the matrix $F$ with the matrix $C$ to obtain an updated third tensor factor $Z$ (as shown in equation 7).

$$B_{bjk} = \sum_i X_{bi} A_{ijk} \ ..... \ (2)$$

$$C_{bk} = \sum_i Y_{bj} B_{bjk} \ ..... \ (3)$$

$$D = X^T X \ ..... \ (4)$$

$$E = Y^T Y \ ..... \ (5)$$

$$F = DE \ ..... \ (6)$$

$$Z = F^{-1}C \ \dots \ (7)$$

Once the third tensor factor $Z$ is updated, the first tensor factor $X$ is updated in a similar way based on the second tensor factor $Y$ and the updated third tensor factor $Z$. More particularly, $X$ is updated by: multiplying transpose of the second tensor factor $Y$ along the first direction of $A$ to obtain a matrix $B'$ (as shown in equation 8); multiplying the matrix $B'$ with transpose of the updated third tensor factor $Z$ along a second direction to obtain a matrix $C'$ (as shown in equation 9); determining product of the second tensor factor $Y$ with transpose of $Y$ as matrix $D'$ (as shown in equation 10) and product of the updated third tensor factor $Z$ with transpose of $Z$ as matrix $E'$ (as shown in equation 11); performing Hadamard product of the matrix $D'$ and the matrix $E'$ to obtain a matrix $F'$ (as shown in equation 12); and multiplying inverse of the matrix $F'$ with the matrix $C'$ to obtain the updated first tensor factor $X$ (as shown in equation 13).

$$B'_{bjk} = \sum_i Y_{bj} A_{ijk} \ \dots \ (8)$$

$$C'_{bk} = \sum_j Z_{bk} B'_{bjk} \ \dots \ (9)$$

$$D' = Y^T Y \ \dots \ (10)$$

$$E' = Z^T Z \ \dots \ (11)$$

$$F' = D'E' \ \dots \ (12)$$

$$X = F'^{-1}C' \ \dots \ (13)$$

Once the third and first tensor factors are updated, the second tensor factor $Y$ is updated in a similar way based on the updated first tensor factor $X$ and the updated third tensor factor $Z$. More particularly, $Y$ is updated by: multiplying transpose of the updated first tensor factor $X$ along the first direction of $A$ to obtain a matrix $B''$ (as shown in equation 14); multiplying the matrix $B''$ with transpose of the updated third tensor factor $Z$ along a second direction to obtain a matrix $C''$ (as shown in equation 15); determining product of the updated first tensor factor $X$ with transpose of $X$ as matrix $D''$ (as shown in equation 16) and product of the updated third tensor factor $Z$ with transpose of $Z$ as matrix $E''$ (as shown in equation 17); computing Hadamard product of the matrix $D''$ and the matrix $E''$ to obtain a matrix $F''$ (as shown in equation 18); and multiplying inverse of the matrix $F''$ with the matrix $C''$ to obtain the updated second tensor factor $Y$ (as shown in equation 19).

$$B''_{bjk} = \sum_i X_{bi} A_{ijk} \ \dots \ (14)$$

$$C''_{bk} = \sum_i Z_{ik} B''_{bjk} \ \dots \ (15)$$

$$D'' = X^T X \ \dots \ (16)$$

$$E'' = Z^T Z \ \dots \ (17)$$

$$F'' = D''E'' \ \dots \ (18)$$

$$Y = F''^{-1}C'' \ \dots \ (19)$$

Finally, updating a) the third tensor factor $Z$, b) the first tensor factor $X$, and c) the second tensor factor $Y$, iteratively until a measured error value (as shown in equation 20) between i) the electronic integral $A$ and ii) a tensor factorized representation comprising the updated first tensor factor, the updated second tensor factor, and the updated third tensor factor is less than a predefined threshold value. The tensor factorized representation obtained after iterative updation is the tensor factorized representation of the electronic integral $A$.

$$Error = \sum_{ijk} \left| A_{ijk} - \sum_{a} X_{ia} Y_{ja} Z_{ka} \right|^2 \;\;\ldots\; (20)$$

Once the tensor factorization is performed, at step 206 of the method 200, the one or more GPUs are configured to determine a density matrix of the molecule by using a Hartree-Fock calculation based on the tensor factorized representation of the one or more electronic integrals. The density matrix is determined by iteratively performing a plurality of steps until a convergence criterion is satisfied. The convergence criterion specifies that norm of difference between a density matrix obtained at a previous iteration and an updated density matrix obtained at a current iteration converges to a value below a predefined threshold convergence error. The plurality of steps performed at each iteration comprises: computing a direct matrix (alternately referred as J matrix) and a hybrid matrix (alternately referred as K matrix) using the tensor factorized representation; constructing a Fock matrix from the direct matrix and the hybrid matrix; performing direct inversion of iterative subspace correction on the Fock matrix; obtaining Kohn Sham Orbitals by performing diagonalization of the Fock matrix after performing the direct inversion of iterative subspace correction; and determining an updated density matrix from the Kohn Sham Orbitals. In an embodiment, diagonalization of the Fock matrix is performed using method disclosed in Indian patent application number 202321061415. In an embodiment, Pulay mixing is performed on the density matrix obtained at previous iteration and the updated density matrix. If the updated density matrix satisfies the convergence criteria, then the iteration is stopped, and Kohn Sham Orbitals based on which the updated density matrix is determined are stored.

Once the density matrix is determined, at step 208 of the method 200, the one or more GPUs are configured to perform multiconfigurational Hamiltonian downfolding on orbitals of the molecule based on the density matrix. A Hamiltonian comprising a molecular orbital representation of the molecule is determined based on Kohn Sham Orbitals corresponding to the density matrix (stored at previous step) and the tensor factorized representation of the one or more electronic integrals. The Hamiltonian is determined according to equation 21, wherein $c_{l\sigma}/c_{l\sigma}^{\dagger}$ are annhiliation and creation operators in the atomic orbital basis, $C$ is the Kohn Sham Orbitals (represented as a rotation matrix) that transforms atomic orbitals to molecular orbitals, $f_{i\sigma}^{\dagger}$ is a creation operator for $i^{th}$ molecular orbital with spin state $\sigma$. Similarly, $f_{i\sigma}$ is an annihilation operator for $i^{th}$ molecular orbital with spin state $\sigma$. Orbital indices denoted by $p, q, r, s...$ span over all spin orbitals, indices denoted by $a, b, c... i,j, k, l. . m, n$ span over all spin orbitals except the outermost orbital, the $N^{th}$ orbital here.

$$H_{(N)} = \sum_{pq,\sigma} h_{pq}^{1,\sigma,(N)} f_{p\sigma}^{\dagger} f_{q\sigma} +$$

$$\sum_{a,b,pqrs,\sigma\sigma'} X_{a,p\sigma} X_{a,q\sigma'} M_{ab} X_{b,r\sigma'} X_{b,s\sigma} f_{p\sigma}^{\dagger} f_{q\sigma'}^{\dagger} f_{r\sigma'} f_{s\sigma}, \, f_{p\sigma} = \sum_{l} C_{pl} \, c_{l\sigma} \;\;\ldots\; (21)$$

Once the Hamiltonian is determined, Hamiltonian downfolding for each of a plurality of virtual orbitals in the Hamiltonian is performed iteratively, starting from highest energy virtual orbital ($N^{th}$ orbital) to be decoupled. The iterative process comprises defining a P space comprising configuration of the virtual orbital to be decoupled among the plurality of virtual orbitals and a Q space that is complementary of the P space. The P space and Q space projection operators are mathematically defined according to equations 22 and 23.

$$P_{(N)} = (1 - \hat{n}_{N\uparrow})(1 - \hat{n}_{N\downarrow}) \;\;\ldots\; (22)$$

$$Q_{(N)} = \hat{n}_{N\uparrow} + \hat{n}_{N\downarrow} + \hat{n}_{N\uparrow}\hat{n}_{N\downarrow} \;\;\ldots\; (23)$$

Further, a generator is defined with a plurality of terms comprising: i) a first term associated with a singles excitation cluster that scatters electrons from the virtual orbital to be decoupled to remaining virtual orbitals from among the plurality of virtual orbitals, wherein a coefficient of the first term is a one rank tensor, ii) a second term associated with a paired doubles excitation cluster that scatters two electrons to any other two virtual orbitals from among the plurality of virtual orbitals, wherein the two electrons includes one electron from the virtual orbital to be decoupled and another electron from at least one other virtual orbital from among the plurality of virtual orbitals, wherein coefficient of the second term is a two-rank tensor, and iii) a third term associated with an unpaired doubles excitation cluster that scatters two electrons to any other two virtual orbitals from among the plurality of virtual orbitals, wherein the two electrons includes one electron from the virtual orbital to be decoupled and another electron from at least one other virtual orbital from among the plurality of virtual orbitals, wherein coefficient of the third term is a three rank tensor factorized into associated tensor factorized representation. The generator is mathematically represented by equation 24.

$$\eta_{(N)} = \sum_{j\sigma} t_j^{1,\sigma}(1 - \widehat{n_{N-\sigma}})f_{N\sigma}^\dagger f_{j\sigma} + \sum_{kl} t_{kl}^2 f_{N\uparrow}^\dagger f_{N\downarrow}^\dagger f_{k\downarrow} f_{l\uparrow} +$$

$$\sum_{aikl} A_{ia} A_{ka} B_{la} f_{N\uparrow}^\dagger f_{i\downarrow}^\dagger f_{k\downarrow} f_{l\uparrow} \ \dots\dots (24)$$

The generator accounts for all possible singles and doubles excitations involving the $N^{th}$ virtual orbital. In equation 24, $t_{i,\sigma}^{1,(N)}$, $A_{ia}A_{ka}B_{la}$ and $t_{ij}^{3,(N)}$ denote singles, mixed-doubles (excitations to only one $N^{th}$ virtual orbital) and paired-doubles (excitations to both $N^{th}$ virtual orbitals) excitation amplitudes respectively. From equation 24 it can be observed that $\eta_{(N)}$ is nilpotent with degree 2, i.e, $(\eta_{(N)})^2 = 0$, $(\eta_{(N)})^3 = 0$ ..., and the polynomial expansion of the similarity transformation $S_{(N)} = e^{\eta(N)}$ naturally terminates at $O(\eta_{(N)})$, where $S_{(N)} = 1 + \eta_{(N)}$.

Once the P space, Q space and the generator are defined, a Bloch equation is constructed in a basis $B$ (as defined by equation 25) of a plurality of multi configurational states coupling the P space and the Q space. The multi configurational states include a plurality of Slater determinants comprising: i) a Hartree Fock (equation 26), ii) a plurality of possible 1-electron excited configurations of an occupied electron to the virtual orbital to be decoupled (equation 27), iii) a plurality of possible 2-electron excited configurations of an occupied electron to the virtual orbital to be decoupled and another virtual orbital from among the plurality of virtual orbitals (equations 28 and 29). The Bloch equation is obtained from similarity transformation of the generator, the Hamiltonian, and an inverse similarity transformation of the generator as shown in equation 30.

$$B = \{|\Phi_{HF}\rangle, |\Phi_{HF,j}^i\rangle, \dots |\Phi_{HF,jN}^{il}\rangle, \dots, |\Phi_{HF,NN}^{il}\rangle\} \ \dots (25)$$

$$\text{Hartree-Fock}|\Phi_{HF}\rangle \ \dots\dots (26)$$

$$\text{Singles}|\Phi_{HF,j}^i\rangle = f_{i\sigma}^\dagger f_{j\sigma}|\Phi_{HF}\rangle \ \dots\dots (27)$$

$$\text{Unpaired Doubles}|\Phi_{HF,jN}^{il}\rangle = f_{l\sigma}^\dagger f_{i\sigma'}^\dagger f_{j\sigma'} f_{N\sigma}|\Phi_{HF}\rangle \ \dots (28)$$

$$\text{Paired Doubles}|\Phi_{HF,NN}^{il}\rangle = f_{l\sigma}^\dagger f_{i\sigma'}^\dagger f_{N\sigma'} f_{N\sigma}|\Phi_{HF}\rangle \ \dots (29)$$

$$\langle B_a|Q_{(N)}S_{(N)}H_N S_{(N)}^{-1}P_{(N)}|B_b\rangle = 0 \ \dots\dots (30)$$

Once the Bloch equation is constructed, Wick ordering (alternatively referred as Normal ordering) of the Bloch equation is performed to compute a plurality of matrix elements with respect to each of the Slater determinants comprised in the Bloch equation. Further, a plurality of residual equations is obtained by setting each of the plurality of matrix elements to zero. The plurality of residual equations are solved to determine coefficients of each of the plurality of terms comprised in the generator. Finally, a downfolded Hamiltonian (given by equation 31) is determined based on the coefficients of each of the plurality of terms comprised in the generator. This process is repeated iteratively for all the virtual orbitals in the Hamiltonian.

$$H_{(N-1)} = P_{(N)}S_{(N)}H_N S_{(N)}^{-1}P_{(N)} \ \dots\dots (31)$$

After all the virtual orbitals have been downfolded, various descriptors such as total energy of ground state, total energy of the many body excitations, self-energy matrix, single point green function are computed from the downfolded Hamiltonian.

USE CASE EXAMPLES

Example 1: Method 200 can be used to compute transition state energies with reactive intermediates, reaction parameters that enable finding drugs with less adverse effects. DLPNO-CCSD (T) level theory with downfolding. Multiconfiguration effects are important to compute reaction enthalpy and activation energy barrier with reactive intermediates.

Example 2: Method 200 can be used to find low energy configuration and binding affinity in multi-protein multi-drug problems. This enables drug relabeling and drug repurposing. Relative binding free energy is at least 100 times smaller than binding energy for multiple protein pockets and not always force fields are available to do multi-Protein analysis. But understanding relative binding free energy is needed for drug reusage or repurpose. Here multi configurational downfolding performed according to method 200 provides that accuracy to use drugs for new targets. The binding free energies computed from method 200 can be correlated with the inhibition constants like $IC_{50}$ from experiments, enabling in-silico simulation of drugs.

Example 3: Method 200 can be used to find low energy crystal polymorphs and also inter molecular torsional energy. This enables finding drug crystal APIs with higher stability which can be taken forward in later stages of drug discovery. Crystal structure polymorphs can be distinguished with Tailored Force fields which need very detailed analysis of the energy landscape. For e.g. there are crystals for which Density Functional Theory (DFT) does not give the right low energy polymorph. Method 200 addresses the multi configurational effects and also long-range dispersion effects. Dispersion effects cannot be added generically in every case. Spin component analysis (singlet, triplet) is required. And there again method 200 is useful.

EXPERIMENTS AND RESULTS

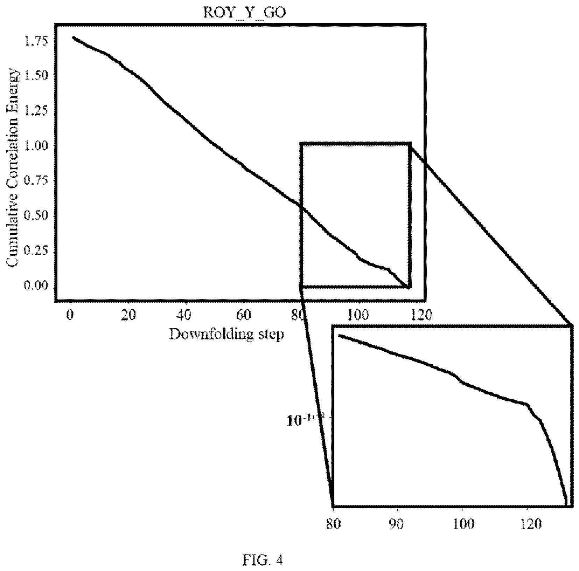

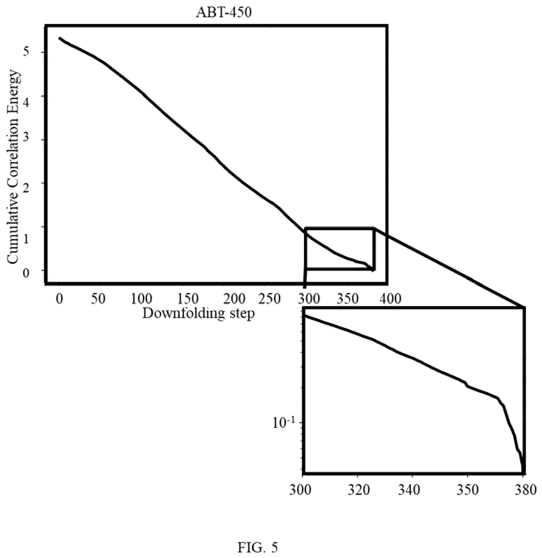

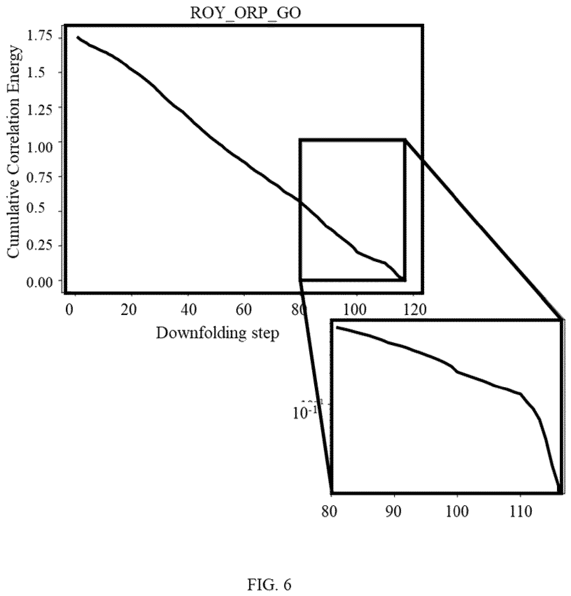

FIGS. 3 to 6 are graphs illustrating cumulative correlation energy at each downfolding step performed by method of FIG. 2 for a plurality of molecules, according to some embodiments of the present disclosure. FIG. 3 illustrates cumulative correlation energy of Headfragment_3 molecule. The head fragment is a molecule with 160 orbitals. Multi configurational downfolding according to method 200 was performed on the molecule and the energy values match against the ones obtained from exact method (performed by physical experiments) within 0.01 Hartree accuracy. FIG. 4 illustrates cumulative correlation energy of geometry optimized ROY_Y conformer molecule which has 190 orbitals. Multi configurational downfolding according to method 200 was performed on the molecule and the energy values match against the ones obtained from exact method within 0.001 Hartree accuracy. Similarly, FIG. 5 illustrates cumulative correlation energy of ABT-450 molecule which has 585 orbitals. Multi configurational downfolding according to method 200 was performed on the molecule and the energy values match against the ones obtained from exact method within 0.05 Hartree accuracy. FIG. 6 illustrates cumulative correlation energy of geometry optimized ROY_ORP conformer molecule which has 190 orbitals. Multi configurational downfolding according to method 200 was performed on the molecule and the energy values match against the ones obtained from exact method within 0.001 Hartree accuracy. Table 1 illustrates comparison of time taken to perform downfolding of the plurality of molecules on Nvidia Tesla V100 64 GB GPU using method 200 and standard state of the art downfolding approach.

Table 1

| Molecule | Method 200 | Standard downfolding approach |
|---|---|---|
| ROY-Y | 10 seconds | 100 seconds |
| ROY-ORP | 12 seconds | 110 seconds |
| ABT450 | 400 seconds | 24,000 seconds |

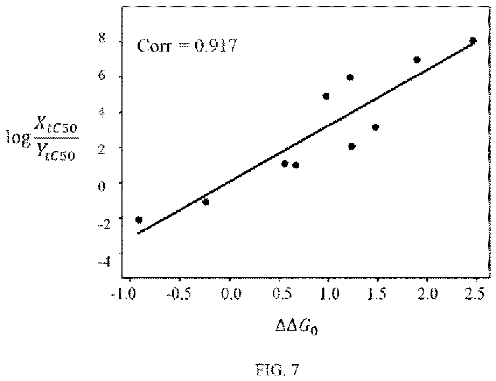

FIG. 7 is a graph illustrating correlation between $IC_{50}$ values of a molecule obtained via method of FIG. 2 (X-axis) and via physical experiments (Y-axis), according to some embodiments of the present disclosure. The molecule considered is a HSP90-alpha protein which has PDB ID=4w7t. It is a protein pocket made of 20 residues with 9 active site residues Asp 93, Thr 184, Met 98, Leu 107, Phe 138, Asn 51, Gly 135, Tyr39, Trp162 and 12 water molecules. Drug molecules 4c,4d,4e,5a and NVPHSP590 were taken and 0K binding Free energy was computed with the protein pocket by performing Geometry Optimization (GO) using multi configurational downfolding of method 200 (alternatively referred as Multi Reference Coupled Cluster Singles Doubles Triples or MRCCSD). Each of the computations for pocket-ligand pairs involved more than 140 atoms and were done on a single Tesla v100 32GB GPU, 64 GB CPU. Totally five 140 heavy atom MRCCSD computations were done for pocket +ligand for 80 GO step, one 100 heavy atom MRCCSD computations were done for pocket for 80 GO steps, five 40 heavy atom MRCCSD computations were done for ligand for 30 GO steps, each 140 heavy atom MRCCSD computations were done for pocket +ligand for 80 GO step were run on the hardware for 85 mins (i.e. one GO step with downfolding takes 1.07 minutes). The total time taken was 5 hours. The Y-axis are experimental values of logarithm of ratio of $IC_{50}$ values for pairs of drug ligands that binding with a kinase pdb-id 4w7t. The X-axis is relative zero temperature Gibbs Binding Free energy computed from method 200.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, non-volatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A quantum simulation method (200) for multiconfigurational downfolding of molecules, performed by a system comprising one or more Central Processing Units (CPUs) and one or more Graphical Processing Units (GPUs), the quantum simulation method comprising:

   obtaining (202), via the one or more CPUs, a configuration of a molecule;
   iteratively estimating (204), via the one or more GPUs, tensor factorized representation of one or more electronic integrals (A) of the molecule, by:

partially computing (204A) the one or more electronic integrals on the fly based on the configuration of the molecule; and

performing (204B) tensor factorization of each of the one or more partially computed electronic integrals to obtain a tensor factorized representation of each of the one or more electronic integrals, wherein the tensor factorized representation comprises a set of tensor factors;

determining (206), via the one or more GPUs, a density matrix of the molecule by using a Hartree-Fock calculation based on the tensor factorized representation of the one or more electronic integrals; and

performing (208), via the one or more GPUs, multi configurational Hamiltonian downfolding on orbitals of the molecule based on the density matrix.

2. The method as claimed in claim 1, wherein the configuration of the molecule comprises one of: atomic coordinates, lattice vectors, and basis vectors.

3. The method as claimed in claim 1, wherein performing the tensor factorization of each of the one or more electronic integrals $A$ to obtain the tensor factorized representation of each of the one or more electronic integrals comprises:

initializing a first tensor factor $X$, a second tensor factor $Y$, and a third tensor factor $Z$;

updating the third tensor factor $Z$, by:

multiplying transpose of the first tensor factor $X$ along a first direction of $A$ to obtain a matrix $B$;

multiplying the matrix $B$ with transpose of the second tensor factor $Y$ along a second direction to obtain a matrix $C$;

determining product of the first tensor factor $X$ with transpose of $X$ as matrix $D$ and product of the second tensor factor $Y$ with transpose of $Y$ as matrix $E$;

performing Hadamard product of the matrix $D$ and the matrix $E$ to obtain a matrix $F$; and

multiplying inverse of the matrix $F$ with the matrix $C$ to obtain an updated third tensor factor $Z$;

updating the first tensor factor $X$ by:

multiplying transpose of the second tensor factor $Y$ along the first direction of $A$ to obtain a matrix $B'$;

multiplying the matrix $B'$ with transpose of the updated third tensor factor $Z$ along a second direction to obtain a matrix $C'$;

determining product of the second tensor factor $Y$ with transpose of $Y$ as matrix $D'$ and product of the updated third tensor factor $Z$ with transpose of $Z$ as matrix $E'$;

performing Hadamard product of the matrix $D'$ and the matrix $E'$ to obtain a matrix $F'$; and

multiplying inverse of the matrix $F'$ with the matrix $C'$ to obtain the updated first tensor factor $X$;

updating the second tensor factor $Y$ by:

multiplying transpose of the updated first tensor factor $X$ along the first direction of $A$ to obtain a matrix $B''$;

multiplying the matrix $B''$ with transpose of the updated third tensor factor $Z$ along a second direction to obtain a matrix $C''$;

determining product of the updated first tensor factor $X$ with transpose of $X$ as matrix $D''$ and product of the updated third tensor factor $Z$ with transpose of $Z$ as matrix $E''$;

computing Hadamard product of the matrix $D''$ and the matrix $E''$ to obtain a matrix $F''$; and

multiplying inverse of the matrix $F''$ with the matrix $C''$ to obtain the updated first tensor factor $Z$;

updating a) the third tensor factor $Z$, b) the first tensor factor $X$, and c) the second tensor factor $Y$, iteratively until a measured error value between i) the electronic integral $A$ and ii) a tensor factorized representation comprising the updated first tensor factor, the updated second tensor factor, and the updated third tensor factor is less than a predefined threshold value, wherein the tensor factorized representation obtained after iterative updation is the tensor factorized representation of the electronic integral $A$.

4. The method as claimed in claim 1, wherein determining the density matrix of the molecule by using the Hartree-Fock calculations comprises iteratively performing a plurality of steps until a convergence criterion is satisfied, wherein the convergence criterion specifies that norm of difference between a density matrix obtained at a previous iteration and an updated density matrix obtained at a current iteration converges to a value below a predefined threshold convergence error, and wherein the plurality of steps comprises:

computing a direct matrix (J matrix) and a hybrid matrix (K matrix) using the tensor factorized representation;
constructing a Fock matrix from the direct matrix and the hybrid matrix;
performing direct inversion of iterative subspace correction on the Fock matrix;
obtaining Kohn Sham Orbitals by performing diagonalization of the Fock matrix after performing the direct inversion of iterative subspace correction; and
determining an updated density matrix from the Kohn Sham Orbitals.

5. The method as claimed in claim 1, wherein performing the multiconfigurational Hamiltonian downfolding on orbitals of the molecule comprises:

determining a Hamiltonian comprising a molecular orbital representation of the molecule based on Kohn Sham Orbitals corresponding to the density matrix and the tensor factorized representation of the one or more electronic integrals; and
iteratively performing Hamiltonian downfolding for each of a plurality of virtual orbitals in the Hamiltonian, starting from highest energy virtual orbital to be decoupled, by:

defining a P space comprising configuration of the virtual orbital to be decoupled among the plurality of virtual orbitals and a Q space that is complementary of the P space;
defining a generator with a plurality of terms comprising: i) a first term associated with a singles excitation cluster that scatters electrons from the virtual orbital to be decoupled to remaining virtual orbitals from among the plurality of virtual orbitals, wherein a coefficient of the first term is a one rank tensor, ii) a second term associated with a paired doubles excitation cluster that scatters two electrons to any other two virtual orbitals from among the plurality of virtual orbitals, wherein the two electrons includes one electron from the virtual orbital to be decoupled and another electron from at least one other virtual orbital from among the plurality of virtual orbitals, wherein coefficient of the second term is a two-rank tensor, and iii) a third term associated with an unpaired doubles excitation cluster that scatters two electrons to any other two virtual orbitals from among the plurality of virtual orbitals, wherein the two electrons includes one electron from the virtual orbital to be decoupled and another electron from at least one other virtual orbital from among the plurality of virtual orbitals, wherein coefficient of the third term is a three rank tensor factorized into associated tensor factorized representation;
constructing a Bloch equation in a basis of a plurality of multi configurational states coupling the P space and the Q space, wherein the multi configurational states include a plurality of Slater determinants comprising: i) a Hartree Fock, ii) a plurality of possible 1-electron excited configurations of an occupied electron to the virtual orbital to be decoupled, iii) a plurality of possible 2-electron excited configurations of an occupied electron to the virtual orbital to be decoupled and another virtual orbital from among the plurality of virtual orbitals, wherein the Bloch equation is obtained from similarity transformation of the generator, the Hamiltonian, and an inverse similarity transformation of the generator;
performing Wick ordering of the Bloch equation to compute a plurality of matrix elements with respect to each of the Slater determinants comprised in the Bloch equation;
obtaining a plurality of residual equations by setting each of the plurality of matrix elements to zero;
solving the plurality of residual equations to determine coefficients of each of the plurality of terms comprised in the generator; and
determining a downfolded Hamiltonian based on the coefficients of each of the plurality of terms comprised in the generator.

6. A system (100), comprising:

a memory (102) storing instructions;
one or more Input/Output (I/O) interfaces (106); and
one or more hardware processors (104) coupled to the memory (102) via the one or more communication interfaces (106), wherein the one or more hardware processors (104) comprise one or more Central Processing Units (CPUs) and one or more Graphical Processing Units (GPUs), wherein the one or more hardware processors are configured by the instructions to:

obtain, via the one or more CPUs, a configuration of a molecule;

iteratively estimate, via the one or more GPUs, tensor factorized representation of one or more electronic integrals (A) of the molecule, by:

partially compute the one or more electronic integrals on the fly based on the configuration of the molecule; and

perform tensor factorization of each of the one or more partially computed electronic integrals to obtain a tensor factorized representation of each of the one or more electronic integrals, wherein the tensor factorized representation comprises a set of tensor factors;

determine, via the one or more GPUs, a density matrix of the molecule by using a Hartree-Fock calculation based on the tensor factorized representation of the one or more electronic integrals; and

perform, via the one or more GPUs, multi configurational Hamiltonian downfolding on orbitals of the molecule based on the density matrix.

7. The system as claimed in claim 6, wherein the configuration of the molecule comprises one of: atomic coordinates, lattice vectors, and basis vectors.

8. The system as claimed in claim 6, wherein performing the tensor factorization of each of the one or more electronic integrals $A$ to obtain the tensor factorized representation of each of the one or more electronic integrals comprises:

initializing a first tensor factor $X$, a second tensor factor $Y$, and a third tensor factor $Z$;

updating the third tensor factor $Z$, by:

multiplying transpose of the first tensor factor $X$ along a first direction of $A$ to obtain a matrix $B$;

multiplying the matrix $B$ with transpose of the second tensor factor $Y$ along a second direction to obtain a matrix $C$;

determining product of the first tensor factor $X$ with transpose of $X$ as matrix $D$ and product of the second tensor factor $Y$ with transpose of $Y$ as matrix $E$;

performing Hadamard product of the matrix $D$ and the matrix $E$ to obtain a matrix $F$; and

multiplying inverse of the matrix $F$ with the matrix $C$ to obtain an updated third tensor factor $Z$;

updating the first tensor factor $X$ by:

multiplying transpose of the second tensor factor $Y$ along the first direction of $A$ to obtain a matrix $B'$;

multiplying the matrix $B'$ with transpose of the updated third tensor factor $Z$ along a second direction to obtain a matrix $C'$;

determining product of the second tensor factor $Y$ with transpose of $Y$ as matrix $D'$ and product of the updated third tensor factor $Z$ with transpose of $Z$ as matrix $E'$;

performing Hadamard product of the matrix $D'$ and the matrix $E'$ to obtain a matrix $F'$; and

multiplying inverse of the matrix $F'$ with the matrix $C'$ to obtain the updated first tensor factor $X$;

updating the second tensor factor $Y$ by:

multiplying transpose of the updated first tensor factor $X$ along the first direction of $A$ to obtain a matrix $B''$;

multiplying the matrix $B''$ with transpose of the updated third tensor factor $Z$ along a second direction to obtain a matrix $C''$;

determining product of the updated first tensor factor $X$ with transpose of $X$ as matrix $D''$ and product of the updated third tensor factor $Z$ with transpose of $Z$ as matrix $E''$;

computing Hadamard product of the matrix $D''$ and the matrix $E''$ to obtain a matrix $F''$; and

multiplying inverse of the matrix $F''$ with the matrix $C''$ to obtain the updated first tensor factor $Z$;

updating a) the third tensor factor $Z$, b) the first tensor factor $X$, and c) the second tensor factor $Y$, iteratively until a measured error value between i) the electronic integral $A$ and ii) a tensor factorized representation comprising the updated first tensor factor, the updated second tensor factor, and the updated third tensor factor is less than a predefined threshold value, wherein the tensor factorized representation obtained after iterative updation is the tensor factorized representation of the electronic integral $A$.

9. The system as claimed in claim 6, wherein determining the density matrix of the molecule by using the Hartree-Fock calculations comprises iteratively performing a plurality of steps until a convergence criterion is satisfied, wherein the convergence criterion specifies that norm of difference between a density matrix obtained at a previous iteration and an updated density matrix obtained at a current iteration converges to a value below a predefined threshold convergence error, and wherein the plurality of steps comprises:

computing a direct matrix (J matrix) and a hybrid matrix (K matrix) using the tensor factorized representation;
constructing a Fock matrix from the direct matrix and the hybrid matrix;
performing direct inversion of iterative subspace correction on the Fock matrix;
obtaining Kohn Sham Orbitals by performing diagonalization of the Fock matrix after performing the direct inversion of iterative subspace correction; and
determining an updated density matrix from the Kohn Sham Orbitals.

10. The system as claimed in claim 6, wherein performing the multiconfigurational Hamiltonian downfolding on orbitals of the molecule comprises:

determining a Hamiltonian comprising a molecular orbital representation of the molecule based on Kohn Sham Orbitals corresponding to the density matrix and the tensor factorized representation of the one or more electronic integrals; and
iteratively performing Hamiltonian downfolding for each of a plurality of virtual orbitals in the Hamiltonian, starting from highest energy virtual orbital to be decoupled, by:

defining a P space comprising configuration of the virtual orbital to be decoupled among the plurality of virtual orbitals and a Q space that is complementary of the P space;
defining a generator with a plurality of terms comprising: i) a first term associated with a singles excitation cluster that scatters electrons from the virtual orbital to be decoupled to remaining virtual orbitals from among the plurality of virtual orbitals, wherein a coefficient of the first term is a one rank tensor, ii) a second term associated with a paired doubles excitation cluster that scatters two electrons to any other two virtual orbitals from among the plurality of virtual orbitals, wherein the two electrons includes one electron from the virtual orbital to be decoupled and another electron from at least one other virtual orbital from among the plurality of virtual orbitals, wherein coefficient of the second term is a two-rank tensor, and iii) a third term associated with an unpaired doubles excitation cluster that scatters two electrons to any other two virtual orbitals from among the plurality of virtual orbitals, wherein the two electrons includes one electron from the virtual orbital to be decoupled and another electron from at least one other virtual orbital from among the plurality of virtual orbitals, wherein coefficient of the third term is a three rank tensor factorized into associated tensor factorized representation;
constructing a Bloch equation in a basis of a plurality of multi configurational states coupling the P space and the Q space, wherein the multi configurational states include a plurality of Slater determinants comprising: i) a Hartree Fock, ii) a plurality of possible 1-electron excited configurations of an occupied electron to the virtual orbital to be decoupled, iii) a plurality of possible 2-electron excited configurations of an occupied electron to the virtual orbital to be decoupled and another virtual orbital from among the plurality of virtual orbitals, wherein the Bloch equation is obtained from similarity transformation of the generator, the Hamiltonian, and an inverse similarity transformation of the generator;
performing Wick ordering of the Bloch equation to compute a plurality of matrix elements with respect to each of the Slater determinants comprised in the Bloch equation;
obtaining a plurality of residual equations by setting each of the plurality of matrix elements to zero;
solving the plurality of residual equations to determine coefficients of each of the plurality of terms comprised in the generator; and
determining a downfolded Hamiltonian based on the coefficients of each of the plurality of terms comprised in the generator.

11. One or more non-transitory machine-readable information storage mediums comprising one or more instructions which when executed by one or more hardware processors comprising one or more Central Processing Units (CPUs) and one or more Graphical Processing Units (GPUs) cause:

obtaining, via the one or more CPUs, a configuration of a molecule;
iteratively estimating, via the one or more GPUs, tensor factorized representation of one or more electronic integrals (A) of the molecule, by:

partially computing the one or more electronic integrals on the fly based on the configuration of the molecule; and

performing tensor factorization of each of the one or more partially computed electronic integrals to obtain a tensor factorized representation of each of the one or more electronic integrals, wherein the tensor factorized representation comprises a set of tensor factors;

determining, via the one or more GPUs, a density matrix of the molecule by using a Hartree-Fock calculation based on the tensor factorized representation of the one or more electronic integrals; and

performing, via the one or more GPUs, multi configurational Hamiltonian downfolding on orbitals of the molecule based on the density matrix.

12. The one or more non-transitory machine-readable information storage mediums as claimed in claim 11, wherein the configuration of the molecule comprises one of: atomic coordinates, lattice vectors, and basis vectors.

13. The one or more non-transitory machine-readable information storage mediums as claimed in claim 11, wherein performing the tensor factorization of each of the one or more electronic integrals $A$ to obtain the tensor factorized representation of each of the one or more electronic integrals comprises:

initializing a first tensor factor $X$, a second tensor factor $Y$, and a third tensor factor $Z$;
updating the third tensor factor $Z$, by:

multiplying transpose of the first tensor factor $X$ along a first direction of $A$ to obtain a matrix $B$;
multiplying the matrix $B$ with transpose of the second tensor factor $Y$ along a second direction to obtain a matrix $C$;
determining product of the first tensor factor $X$ with transpose of $X$ as matrix $D$ and product of the second tensor factor $Y$ with transpose of $Y$ as matrix $E$;
performing Hadamard product of the matrix $D$ and the matrix $E$ to obtain a matrix $F$; and
multiplying inverse of the matrix $F$ with the matrix $C$ to obtain an updated third tensor factor $Z$;

updating the first tensor factor $X$ by:

multiplying transpose of the second tensor factor $Y$ along the first direction of $A$ to obtain a matrix $B'$;
multiplying the matrix $B'$ with transpose of the updated third tensor factor $Z$ along a second direction to obtain a matrix $C'$;
determining product of the second tensor factor Y with transpose of $Y$ as matrix $D'$ and product of the updated third tensor factor $Z$ with transpose of $Z$ as matrix $E'$;
performing Hadamard product of the matrix $D'$ and the matrix $E'$ to obtain a matrix $F'$; and
multiplying inverse of the matrix $F'$ with the matrix $C'$ to obtain the updated first tensor factor $X$;

updating the second tensor factor $Y$ by:

multiplying transpose of the updated first tensor factor $X$ along the first direction of $A$ to obtain a matrix $B''$;
multiplying the matrix $B''$ with transpose of the updated third tensor factor $Z$ along a second direction to obtain a matrix $C''$;
determining product of the updated first tensor factor $X$ with transpose of $X$ as matrix $D''$ and product of the updated third tensor factor $Z$ with transpose of $Z$ as matrix $E''$;
computing Hadamard product of the matrix $D''$ and the matrix $E''$ to obtain a matrix $F''$; and
multiplying inverse of the matrix $F''$ with the matrix $C''$ to obtain the updated first tensor factor $Z$;

updating a) the third tensor factor $Z$, b) the first tensor factor $X$, and c) the second tensor factor $Y$, iteratively until a measured error value between i) the electronic integral $A$ and ii) a tensor factorized representation comprising the updated first tensor factor, the updated second tensor factor, and the updated third tensor factor is less than a predefined threshold value, wherein the tensor factorized representation obtained after iterative updation is the tensor factorized representation of the electronic integral $A$.

14. The one or more non-transitory machine-readable information storage mediums as claimed in claim 11, wherein determining the density matrix of the molecule by using the Hartree-Fock calculations comprises iteratively performing a plurality of steps until a convergence criterion is satisfied, wherein the convergence criterion specifies that norm of difference between a density matrix obtained at a previous iteration and an updated density matrix obtained at a current iteration converges to a value below a predefined threshold convergence error, and wherein the plurality of steps comprises:

computing a direct matrix (J matrix) and a hybrid matrix (K matrix) using the tensor factorized representation;
constructing a Fock matrix from the direct matrix and the hybrid matrix;
performing direct inversion of iterative subspace correction on the Fock matrix;
obtaining Kohn Sham Orbitals by performing diagonalization of the Fock matrix after performing the direct inversion of iterative subspace correction; and
determining an updated density matrix from the Kohn Sham Orbitals.

15. The one or more non-transitory machine-readable information storage mediums as claimed in claim 11, wherein performing the multi configurational Hamiltonian downfolding on orbitals of the molecule comprises:

determining a Hamiltonian comprising a molecular orbital representation of the molecule based on Kohn Sham Orbitals corresponding to the density matrix and the tensor factorized representation of the one or more electronic integrals; and
iteratively performing Hamiltonian downfolding for each of a plurality of virtual orbitals in the Hamiltonian, starting from highest energy virtual orbital to be decoupled, by:

defining a P space comprising configuration of the virtual orbital to be decoupled among the plurality of virtual orbitals and a Q space that is complementary of the P space;
defining a generator with a plurality of terms comprising: i) a first term associated with a singles excitation cluster that scatters electrons from the virtual orbital to be decoupled to remaining virtual orbitals from among the plurality of virtual orbitals, wherein a coefficient of the first term is a one rank tensor, ii) a second term associated with a paired doubles excitation cluster that scatters two electrons to any other two virtual orbitals from among the plurality of virtual orbitals, wherein the two electrons includes one electron from the virtual orbital to be decoupled and another electron from at least one other virtual orbital from among the plurality of virtual orbitals, wherein coefficient of the second term is a two-rank tensor, and iii) a third term associated with an unpaired doubles excitation cluster that scatters two electrons to any other two virtual orbitals from among the plurality of virtual orbitals, wherein the two electrons includes one electron from the virtual orbital to be decoupled and another electron from at least one other virtual orbital from among the plurality of virtual orbitals, wherein coefficient of the third term is a three rank tensor factorized into associated tensor factorized representation;
constructing a Bloch equation in a basis of a plurality of multi configurational states coupling the P space and the Q space, wherein the multi configurational states include a plurality of Slater determinants comprising: i) a Hartree Fock, ii) a plurality of possible 1-electron excited configurations of an occupied electron to the virtual orbital to be decoupled, iii) a plurality of possible 2-electron excited configurations of an occupied electron to the virtual orbital to be decoupled and another virtual orbital from among the plurality of virtual orbitals, wherein the Bloch equation is obtained from similarity transformation of the generator, the Hamiltonian, and an inverse similarity transformation of the generator;
performing Wick ordering of the Bloch equation to compute a plurality of matrix elements with respect to each of the Slater determinants comprised in the Bloch equation;
obtaining a plurality of residual equations by setting each of the plurality of matrix elements to zero;
solving the plurality of residual equations to determine coefficients of each of the plurality of terms comprised in the generator; and
determining a downfolded Hamiltonian based on the coefficients of each of the plurality of terms comprised in the generator.

SYSTEM
100

MEMORY
102

DATABASE
108

HARDWARE
PROCESSOR(S)
104

I/O INTERFACE(S)
106

FIG. 1

200

Obtaining, via the one or more CPUs, a configuration of a molecule — 202

Iteratively estimating, via the one or more GPUs, tensor factorized representation of one or more electronic integrals (A) of the molecule, by: — 204

Partially computing the one or more electronic integrals on the fly based on the configuration of the molecule, wherein each of the one or more electronic integrals is a three-rank tensor — 204A

Performing tensor factorization of each of the one or more electronic integrals to obtain a tensor factorized representation of each of the one or more electronic integrals, wherein the tensor factorized representation comprises a set of tensor factors, and wherein each tensor factor in the set of tensor factors is a two-rank tensor — 204B

Determining, via the one or more GPUs, a density matrix of the molecule by using a Hartree-Fock calculation based on the tensor factorized representation of the one or more electronic integrals — 206

Performing, via the one or more GPUs, multiconfigurational Hamiltonian downfolding on orbitals of the molecule based on the density matrix — 208

FIG. 2

FIG. 3

FIG. 4

FIG. 5

FIG. 6

FIG. 7

Europäisches
Patentamt

European
Patent Office

Office européen
des brevets

# EUROPEAN SEARCH REPORT

**Application Number**

EP 25 17 6269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | RITAM BANERJEE ET AL: "Tensor Factorized Hamiltonian Downfolding To Optimize The Scaling Complexity Of The Electronic Correlations Problem on Classical and Quantum Computers", ARXIV.ORG, CORNELL UNIVERSITY LIBRARY, 201 OLIN LIBRARY CORNELL UNIVERSITY ITHACA, NY 14853, 3 May 2025 (2025-05-03), XP092012121, * the whole document * | 1-15 | INV. G16C10/00 |
| X | BAUMAN NICHOLAS P. ET AL: "Coupled Cluster Downfolding Theory: towards universal many-body algorithms for dimensionality reduction of composite quantum systems in chemistry and materials science", MATERIALS THEORY, vol. 6, no. 1, 7 May 2022 (2022-05-07), XP093320627, ISSN: 2509-8012, DOI: 10.1186/s41313-022-00046-8 * the whole document * | 1-15 | |
| X | DATTA DIPAYAN ET AL: "Accelerating Coupled-Cluster Calculations with GPUs: An Implementation of the Density-Fitted CCSD(T) Approach for Heterogeneous Computing Architectures Using OpenMP Directives", JOURNAL OF CHEMICAL THEORY AND COMPUTATION: JCTC, vol. 19, no. 21, 25 October 2023 (2023-10-25), pages 7640-7657, XP093320738, US ISSN: 1549-9618, DOI: 10.1021/acs.jctc.3c00876 * the whole document * | 1-15 | **TECHNICAL FIELDS SEARCHED (IPC)** <br><br> G16C |

The present search report has been drawn up for all claims

| Place of search | Date of completion of the search | Examiner |
|---|---|---|
| The Hague | 30 September 2025 | Denoual, Matthieu |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421039027

- IN 202321061415